Europäisches Patentamt

European Patent Office

Office européen des brevets

(19)

(11) Numéro de publication: **0 149 383**
**B1**

(12) **FASCICULE DE BREVET EUROPEEN**

(45) Date de publication du fascicule du brevet:
27.01.88

(51) Int. Cl.⁴: **C 12 Q 1/18, G 01 N 33/04**

(21) Numéro de dépôt: **84402549.4**

(22) Date de dépôt: **11.12.84**

(54) **Procédé et dispositif de détection de l'activité d'une substance de type bactériophage sur un micro-organisme ou sur un mélange de micro-organismes lactiques.**

(30) Priorité: **14.12.83 FR 8320014**

(43) Date de publication de la demande:
**24.07.85 Bulletin 85/30**

(45) Mention de la délivrance du brevet:
**27.01.88 Bulletin 88/4**

(84) Etats contractants désignés:
**AT BE CH DE FR GB IT LI LU NL SE**

(56) Documents cités:
**EP - A - 0 005 891**
**FR - A - 1 488 866**
**FR - A - 2 261 517**
**FR - A - 2 347 439**
**US - A - 3 843 456**
**US - A - 4 284 725**

(73) Titulaire: **L'AIR LIQUIDE, SOCIETE ANONYME POUR L'ETUDE ET L'EXPLOITATION DES PROCEDES GEORGES CLAUDE, 75, Quai d'Orsay, F-75321 Paris Cédex 07 (FR)**

(72) Inventeur: **Boussemaer, Jean-Pierre, Lotissement Le Matton, F-38620 Massieu (FR)**

(74) Mandataire: **Bouton Neuvy, Liliane et al, L'Air liquide, Société Anonyme pour L'Etude et L'Exploitation des Procédés Georges Claude 75, Quai d'Orsay, F-75321 Paris Cédex 07 (FR)**

## Description

La présente invention est relative à un procédé de détection de l'activité d'une substance de type bactériophage présente dans les cuves de fromagerie sur un micro-organisme de type bactérie lactique ou sur un mélange de bactéries lactiques, ainsi qu'à un dispositif permettant de mettre en œuvre ce procédé. L'invention s'applique notamment au dépistage préventif des bactériophages en fromagerie.

Dans une fromagerie, on effectue la fermentation du lait dans des cuves au moyen d'un levain réalisé à partir d'un mélange d'un certain nombre de souches bactériennes. Au cours du temps se développent dans les cuves des micro-organismes appelés bactériophages, ou plus simplement phages; la croissance de ces phages suppose celle des bactéries lactiques et est considérablement plus rapide que celle de ces dernières. Les phages sont plus ou moins spécifiques des bactéries lactiques utilisées; à partir d'une certaine concentration d'un type donné de phage dans la cuve, les bactéries correspondantes sont détruites de façon suffisamment importante pour ne plus produire la fermentation attendue. L'accident de fabrication en fromagerie se produit lorsqu'une proportion suffisante des souches est contaminée.

L'activité des bactéries lactiques peut également diminuer pour d'autres raisons; en particulier, elle peut être stoppée par la présence de substances inhibitrices se trouvant dans le lait utilisé ou fabriquées par les bactéries elles-mêmes, ou encore par des antibiotiques présents dans le lait.

On comprend, dans ces conditions, l'intérêt pour les fromagers d'avoir à leur disposition un test permettant de déterminer de façon fiable et préventive la présence de l'un ou l'autre de ces facteurs nuisibles.

Par le brevet US 3 843 456, on connaît la préparation d'une culture standard de bactéries dans un milieu permettant leur survie, mais non leur croissance; et selon EP-A-0 005 891 ce principe est appliqué à la recherche d'antibiotiques.

L'invention a pour but de fournir un test fiable très sensible ayant un caractère préventif et extrêmement simple à utiliser en fromagerie, où il permet d'obtenir une véritable échelle de contamination des mélanges de souches bactériennes lactiques.

A cet effet, l'invention a pour objet un procédé de détection de l'activité d'une substance du type bactériophage présent dans les cuves de fromagerie sur un micro-organisme du type bactérie lactique, caractérisé par la succession d'étapes suivantes:

(a) on prépare dans un récipient un milieu lyophilisé contenant le micro-organisme et une partie d'un milieu de culture de ce micro-organisme;

(b) on prépare une solution contenant le complément dudit milieu de culture;

(c) on ajoute à cette solution ladite substance;

(d) on verse dans le récipient le liquide ainsi obtenu;

(e) on fait incuber le récipient; et

(f) on soumet le récipient à un test de présence du micro-organisme.

Dans un mode de mise en œuvre particulièrement intéressant, le procédé de l'invention est appliqué à la détermination de l'activité de ladite substance sur un mélange de n micro-organismes. Dans ce cas, l'étape (a) décrite ci-dessus est remplacée par l'étape (al) suivante:

(al) on prépare dans chacun de n récipients un milieu lyophilisé contenant un micro-organisme respectif du mélange, ainsi qu'une partie d'un milieu de culture de l'ensemble des micro-organismes; et on applique chacune des étapes (d), (e) et (f) à chacun des récipients.

L'invention a également pour objet un dispositif ou «kit» de mise en œuvre de ce dernier procédé. Ce dispositif comprend:

– un ensemble de portoir de cupules de microtitration réunissant n rangées de m cupules, chaque rangée contenant un milieu lyophilisé qui contient lui-même un micro-organisme respectif dudit mélange;

– 4 (m-1) tubes stériles de prélèvement; et

– 4 m tubes de dilution contenant ladite solution.

Dans un mode de réalisation avantageux, le dispositif suivant l'invention comprend en outre un peigne agitateur dont les dents sont adaptées pour pénétrer simultanément dans tous les récipients d'une même rangée ou d'une même colonne.

Du fait que les cupules doivent à plusieurs reprises être bouchées et débouchées suivant une autre caractéristique avantageuse de l'invention, les rangées de cupules sont munies d'un système d'obturation constitué d'une feuille sur laquelle font saillie vers le bas des alvéoles borgnes en nombre égal à celui des cupules et adaptés pour s'emboîter à force dans ces cupules.

Un exemple de mis en œuvre de l'invention va maintenant être décrit en regard des dessins annexés, sur lesquels:

la fig. 1 est une vue schématique d'un dispositif suivant l'invention destiné à l'étude d'un mélange de bactéries lactiques utilisé en fromagerie;

la fig. 2 est une vue partielle prise en coupe suivant la ligne II-II de la fig. 1;

la fig. 3 est une vue en plan de la plaquette de microtitration de la fig. 1;

la fig. 4 représente un accessoire pouvant être utilisé avec le dispositif de la fig. 1; et

la fig. 5 est un schéma illustrant les diverses étapes du procédé mis en œuvre avec le dispositif des figures 1 à 4.

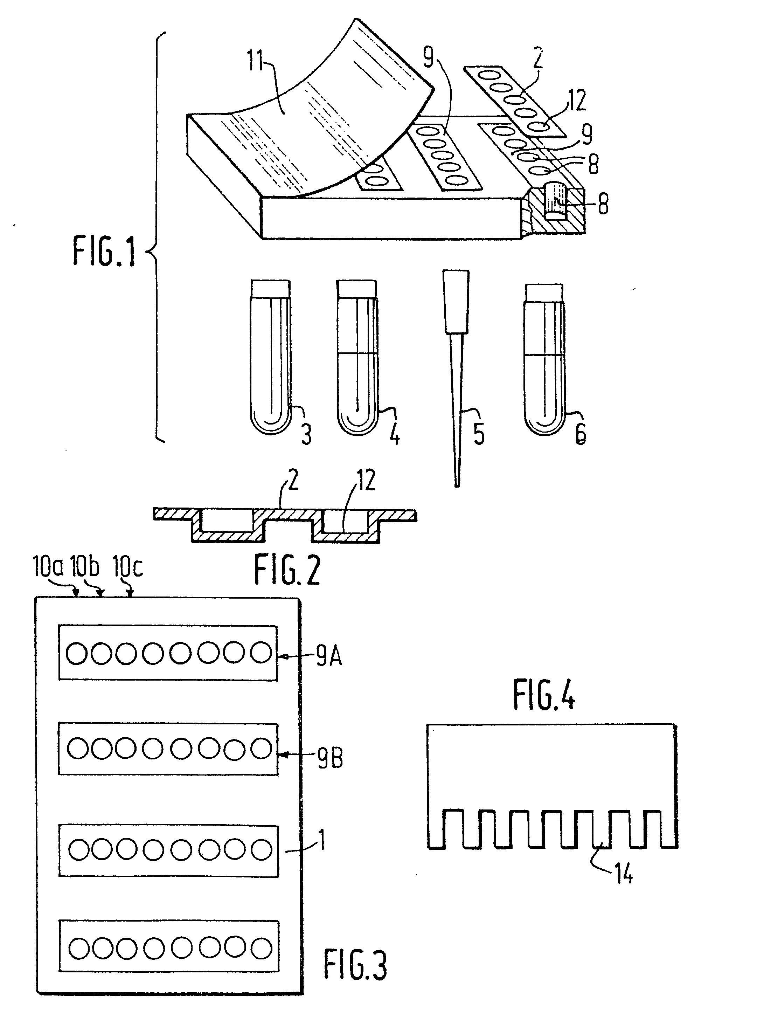

Le procédé qui va être décrit ci-dessous est appliqué à la fabrication de fromages. Il permet de déterminer à tout instant le degré de contamination du mélange de souches bactériennes en cours de fermentation dans une cuve, dans le cas où ce mélange est constitué d'un ensemble de souches bien déterminées. On décrira tout d'abord, en regard des figures 1 à 4, le dispositif ou «kit» permettant cette détermination.

Ce dispositif comprend essentiellement: un ensemble de 3 ou 4 portoirs de cupules de microtitration 1 pourvue d'un couvercle 2; une série de 28 tubes de prélèvement 3; une série de 32 tubes de dilution 4; une série de cônes de prélèvement 5 du commerce, connus sous l'appellation «cônes jaunes», destinés à servir d'embouts interchangeables pour une pipette automatique non représentée; et un tube de réactif 6. A ces éléments peut être adjoint un peigne agitateur 7 représenté à la fig. 4. Un seul tube 3, un seul tube 4 et un seul cône 5 ont été représentés à la fig. 1 dans un but de clarté. Les éléments 1 à 4 sont livrés stériles. Le portoir de cupules 1 est constitué d'une feuille de matière plastique thermoformée (P.V.C.) de forme parallélépipédique qui comporte dans sa face supérieure quatre alvéoles pouvant recevoir chacune un rangée de huit cupules. Les quatre rangées de cupules d'un même portoir contiennent la même souche. On a donc autant de portoirs qu'il y a de souches dans le mélange. Un portoir vide identique aux précédents est destiné à recevoir une rangées de cupules de chaque portoir de manière à constituer un portoir contenant les souches du mélange à tester. En supposant le portoir orienté, en vue en plan comme à la fig. 3 avec ses grands côtés verticaux, chaque rangée horizontale référencée 9A, 9B . . . est affectée à une souche déterminée A, B, . . . du mélange; la 1ère colonne 10a (celle de gauche dans l'exemple représenté) constitue une colonne témoin et chacune des sept autres colonnes 10b, 10c, . . . est affectée à un prélèvement. Le portoir permet donc de tester un à sept échantillons avec un mélange contenant une à quatre souches A, B . . .

Chaque cupule 8 contient une souche conservée sous forme lyophilisée. Plus précisément, cette souche est incorporée à un lyophilisat formant d'une part un protecteur des bactéries vis-à-vis de la lyophilisation et d'autre part une partie d'un milieu de culture de la souche. A titre d'exemple, ce lyophilisat peut être constitué comme suit:

| | | |
|---|---|---|
| glycérophosphate | 75 | g |
| glutamate de Na | 20 | g |
| extrait de levure | 30 | g |
| chlorure de sodium | 12 | g |
| chlorure de calcium | 2,25 | g |
| acétate de sodium | 4,5 | g |
| sulfate de magnésium | 0,6 | g |
| poudre de lait | 62,5 | g |
| H20 qsp | 1000 | ml |

La souche est incorporée à ce milieu avant lyophilisation dans une proportion déterminée variable de 1 à 10% suivant la souche, sous la forme d'une culture de 16 heures sur lait peptoné stérile, coagulée par la souche.

La lyophilisation est effectuée directement dans les portoirs. Lorsque la lyophilisation est terminée, le courvercle 2, qui va être décrit plus en détail ci-dessous, est mis en place sur les rangées de cupules et les portoirs sont scellés par des feuilles métalloplastiques (stratifié constitué d'une couche extérieure métallique et d'une couche intérieure en matière plastique thermosoudée sur le portoir) et conservés à +4°C.

Le courvercle 2 est constitué d'une feuille de matière plastique souple et transparente ayant à peu près les mêmes dimensions qu'une rangée de cupules 9.

Dans cette feuille sont formés huit alvéoles borgnes 12 sensiblement cylindriques, en saillie vers le bas, qui présentent la même disposition et sensiblement le même diamètre que les cupules 8. Lorsque la lyophilisation du portoir 1 est terminée, on réalise un bouchage étanche des cupules 8 en appliquant fermement le couvercle 2 sur la rangée de cupules 9, chaque alvéole 12 s'emboîtant, légèrement à force, dans la cupule correspondante.

La matière plastique du couvercle 2 doit bien entendu, comme celle du portoir 1, présenter sous un faible coût des propriétés physiques et chimiques adaptées aux corps manipulés et aux températures auxquelles elle est soumise, en particulier à un froid de – 30°C. On peut par exemple réaliser la plaquette en polystyrène cristal et le couvercle 2 en polystyrène.

Chacun des huit tubes de dilution 4 contient 3 ml d'un liquide servant d'une part à la réhydratation du lyophilisat contenu dans les cupules 8 d'une colonne (10a, 10b, . . .), d'autre part à la dilution d'un échantillon prélevé au moyen d'un tube 3, et enfin à la complémentation du milieu de culture du lyophilisat. Ce milieu a la composition suivante:

| | | |
|---|---|---|
| extrait de levure | 20 | g |
| lactose | 30 | g |
| H20 qsp | 1 litre | |
| BCP (bromocresolpurple) | 0,02 g | |

Ce milieu possède un pH de 6,5 et est stérilisé à 120°C pendant 20 minutes. Le BCP est un indicateur de pH dont la couleur est pourpre pour les pH supérieurs à 5 environ et jaune pour les pH inférieurs à cette valeur.

Les tubes 6 contiennent un réactif permettant de lire les résultats du test. Ce réactif est une solution stérile de «vert janus», et on verse 20 µl par cupule, le vert janus étant dilué à 0,25 g/l. Ce réactif possède la propriété de virer au rose en présence des bactéries lactiques.

Le peigne 7 est un morceau d'une feuille de matière plastique ayant une forme générale de demi-disque. Sur le diamètre de ce demi-disque font saillie huit dents rectangulaires (14) dont la largeur est inférieure au diamètre des cupules 8.

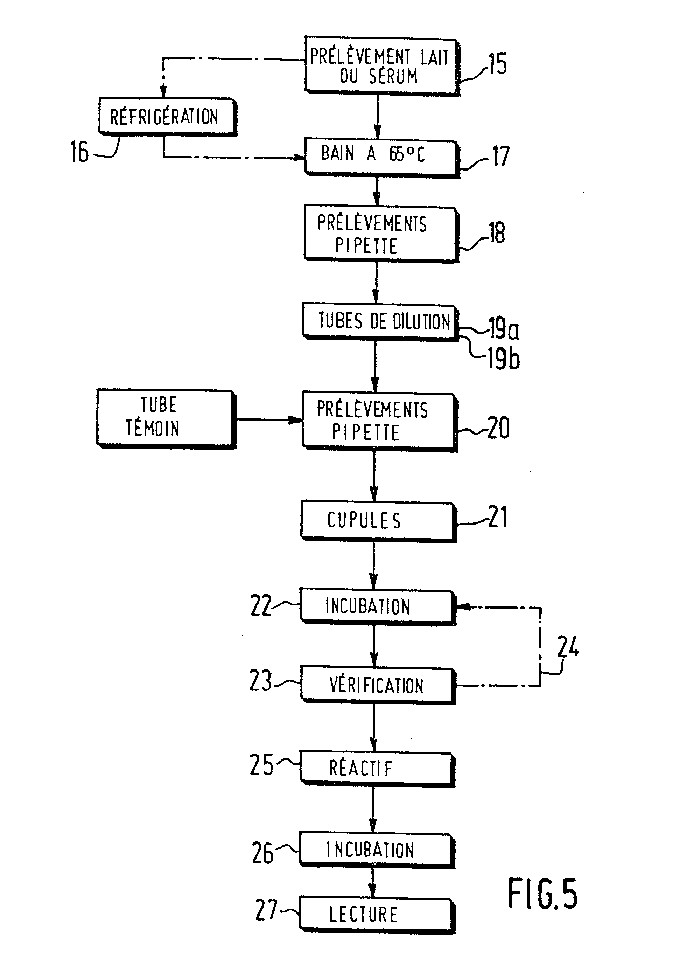

Le dispositif ainsi décrit s'utilise de la manière suivante, illustrée à la fig. 5. L'étude porte sur un lait traité en fromagerie par un levain réalisé, par exemple, avec un mélange des huit souches A, B, . . . on analyse sur ces huit souches les quatre souches principales conservées respectivement dans les huit rangées 9A, 9B, . . . de ce portoir.

Pour des raisons économiques, il est souhaitable d'effectuer le maximum de prélèvement possible (référence 15), c'est-à-dire sept prélèvements. Le nombre de prélèvements journalier dépend des paramètres technologiques de la fromagerie (volume de lait traité, nombre de cuves, etc...) et des buts poursuivis (contrôle de contamination, explication d'un incident constaté, suivi régulier, etc...). Si ce nombre est inférieur à sept, on conserve les échantillons au réfrigérateur ou au congélateur, comme illustré en 16 à la figure 5. Les échantillons sont constitués du lait pasteurisé de départ ou du sérum d'une cuve en cours de fermentation.

Le jour du test, un bain thermostaté est réglé sur 65°C. Les échantillons de sérum, après avoir éventuellement été ramenés à température ambiante, sont placés pendant 7 mn dans ce bain thermostaté (référence 17 de la fig. 5).

Cette opération supprime à la fois les bactéries lactiques et une partie notable des substances inhibitrices, sans détruire les bactériophages.

Dès la sortie du bain, on prélève 170 µl de chaque échantillon au moyen d'une pipette à piston dite «pipette automatique» préréglée sur ce volume et munie d'un cône jaune (référence 18). Ces 170 µl sont versés dans un tube d'eau peptonée 4 (référence 19), que l'on agite. Ainsi, sept tubes 4 reçoivent une quantité dosée d'un échantillon respectif, tandis que le huitième tube 4, qui est un tube témoin, n'en reçoit pas.

Toutes les opérations qui suivent sont effectuées près d'une flamme (bec bunsen . . .) pour assurer la stérilité des manipulations.

Une rangée de cupules est dégagée de chacun des trois ou quatre portoirs. Les trois ou quatre rangées de cupules ainsi dégagées sont disposées dans le portoir vide. Le couvercle de chaque rangée est retiré. La phase suivante 20 consiste à prélever dans chaque tube 4, à l'aide de la même pipette automatique munie d'un nouveau cône jaune 5, que l'on change pour chaque tube, des doses de 170 µl qui sont versées dans les cupules 8 (référence 21). Plus précisément, chaque cupule 8 de la colonne 10a̲ reçoit 170 µl provenant du tube témoin 4, chaque cupule de la deuxième colonne 10b̲ reçoit 170 µl provenant du tube 4 qui contient le premier échantillon, etc... Au cours de cette opération, on prend soin de ne pas toucher le bord des cupules ou les lyophilisats avec le cône de distribution, ceci afin d'éviter les intercontaminations.

Après avoir ainsi distribué tous les échantillons, les couvercles 2 sont remis en place dans leur position initiale. Après quelques minutes, le portoir est agité dans le sens horizontal, en évitant le contact liquide-couvercle, puis il est mis à incuber à 30°C pendant 16 à 20 heures (référence 22). Cette durée est déterminée de manière à permettre aux souches contenues dans la première colonne d'abaisser le pH au-dessous de 5. On vérifie donc (référence 23) que toutes les cupules de la colonne (10a̲) sont devenues jaunes, sinon (référence 24) on prolonge un peu l'incubation.

Ensuite (référence 25), on verse 20 µl de réactif du tube 6 dans chaque cupule, et l'on agite. Pour cela, on peut agiter horizontalement la plaquette, comme précédemment, mais comme les phénomènes microbiologiques sont terminés à ce stade et que tout risque d'intercontamination est par conséquent écarté, il est commode d'utiliser le peigne 14, qui permet d'agiter d'un seul geste toutes les cupules d'une colonne, puis de la colonne suivante, etc... Puis (référence 26), on remet le portoir muni des couvercles 2 à incuber à 30°C pendant environ 45 minutes à 1 heure pour faire réagir le vert janus.

Il ne reste plus qu'à sortir le portoir de l'étude, à retirer le couvercle 2, et à effectuer la lecture (référence 27). Trois cas peuvent se présenter, résultant des considérations suivantes pour chaque souche du mélange:

– si les échantillons de lait conduisent à une couleur rose, ceci signifie que le lait utilisé n'a pas empêché l'activité de la souche. Il ne contient donc pas de substance inhibitrices ou d'antibiotique nuisible à la fermentation par cette souche;

– si les échantillons de lait conduisent à une couleur verte, ce lait contient au contraire des inhibiteurs et des antibiotiques. Ceci se retrouve dans la cuve, de sorte que la souche ne peut agir, même en l'absence de phages;

– si des échantillons de sérum conduisent à une couleur verte, il existe dans la cuve soit des inhibiteurs et/ou des antibiotiques provenant du lait, soit des phages.

Par suite, en prélevant à la fois des échantillons de lait et des échantillons de sérum, on aboutit aux conclusions suivantes:

| Lait | Sérum | Interprétation |
|------|-------|----------------|
| rose | rose | normal |
| vert | vert | inhibieur ou antibiotique dans le lait |
| rose | vert | bactériophages |

En supposant le lait dépourvu d'inhibiteur et d'antibiotique, la proportion de cupules vertes par rapport à l'ensemble des cupules donne la proportion de souches du mélange contaminées par les bactériophages. On peut admettre généralement que jusqu'à 40% de cupules vertes, il n'y a pas de risque à utiliser ce mélange 2 à 3 jours. Par contre, au-dessus de cette proportion, il est indispensable de changer de mélange pour les fabrications suivantes.

Ce test, effectué sur les souches pures (c'est-à-dire isolées) des mélanges utilisés, indique de façon fiable le degré de contamination de ces mélanges. Grâce à sa présentation en kit, peu coûteuse, il est simple à utiliser. La conservation des souches sous forme lyophilisée, à des concentrations bien définies, supprime toute opération de culture bactérienne et de repiquage. Le milieu de culture utilisé assure une très bonne croissance des souches et des phages ainsi qu'une très bonne protection des souches lyophilisées. De plus, le vert janus possède un virage de couleur très net, ce qui rend facile l'interprétation des résultats.

**Revendications**

1. Procédé de détection de l'activité d'une substance du type bactériophage présent dans les cuves de fromagerie sur un micro-organisme du type bactérie lactique, caractérisé par la succession d'étapes suivantes:

(a) on prépare dans un récipient un milieu lyophilisé contenant le micro-organisme et une partie d'un milieu de culture de ce micro-organisme;

(b) on prépare une solution contenant le complément dudit milieu de culture;

(c) on ajoute à cette solution ladite substance;

(d) on verse dans le récipient le liquide ainsi obtenu;

(e) on fait incuber le récipient; et

(f) on soumet le récipient à un test de présence du micro-organisme.

2. Procédé suivant la revendication 1, pour la détermination de l'activité de ladite substance sur un mélange de n micro-organismes, caractérisé en ce que l'étape (a) est remplacée par l'étape (al) suivante:

(al) on prépare dans chacun de n récipients un milieu lyophilisé contenant un micro-organisme respectif du mélange, ainsi qu'une partie d'un milieu de culture de l'ensemble des micro-organismes; et en ce qu'on applique chacune des étapes (d), (e) et (f) à chacun des récipients.

3. Procédé suivant la revendication 1, caractérisé en ce que, dans le cas où ladite substance contient également le micro-organisme ou le mélange de micro-organismes, on effectue, entre les étapes (a) ou (al) d'une part, (c) d'autre part, l'étape supplémentaire suivante:

(g) on soumet ladite substance à un traitement qui en élimine le micro-organisme ou le mélange de micro-organismes; et en ce qu'on remplace l'étape (c) par l'étape suivante:

(cl) on ajoute à ladite solution ladite substance traité par l'étape (g).

4. Procédé suivant la revendication 3, caractérisé en ce que le traitement de l'étape (g) est constitué par un chauffage.

5. Procédé selon la revendication 1, caractérisé en ce que, pour le ou pour chacun des micro-organismes, on prépare, dans un récipient supplémentaire, ledit milieu lyophilisé; on prépare, une solution témoin analogue à ladite solution mais contenant en outre un indicateur de l'activité du ou de chaque micro-organisme; on verse la solution témoin dans ledit récipient supplémentaire; et l'on prolonge l'étape (e) jusqu'à ce que ledit indicateur ait montré une activité prédéterminée du ou de chaque micro-organisme.

6. Dispositif de mise en œuvre d'un procédé suivant la revendication 2, caractérisé en ce qu'il comprend:

– un portoir de cupules de microtitration (1) réunissant <u>n</u> rangées (9A, 9B . . .) de

– m cupules (8), chaque rangée contenant un milieu lyophilisé qui contient lui-même un micro-organisme respectif dudit mélange;

– 4 (m-1) tubes stériles de prélèvement (3); et

– 4 m tubes de dilution (4) contenant ladite solution.

7. Dispositif suivant la revendication 6, caractérisé en ce que chaque rangée (9A, 9B . . .) du portoir de cupules (1) comporte une (m)ème cupule (8) et en ce qu'il est prévu un (m)ème tube de dilution (4) contenant ladite solution, formant tube-témoin est associé aux (m)èmes cupules.

8. Dispositif suivant la revendication 6, caractérisé en ce qu'il comprend en outre un tube (6) d'un indicateur coloré de la présence des micro-organismes du mélange.

9. Dispositif suivant la revendication 6, caractérisé en ce que chaque tube de dilution (4) contient un indicateur coloré de l'activité des micro-organismes du mélange.

10. Dispositif suivant la revendication 6, caractérisé en ce qu'il comprend en outre un peigne agitateur (7) dont les dents (14) sont adaptées pour pénétrer simultanément dans tous les récipients (8) d'une même rangée (9A, 9B, . . .) ou d'une même colonne (10a, 10b, . . .).

11. Dispositif suivant la revendication 6, caractérisé en ce que chaque rangée de cupules (9) est munie d'un couvercle (2) constitué d'une feuille sur laquelle font saillie vers le bas des alvéoles borgnes (12) en nombre égal à celui des cupules (8) et adaptés pour s'emboiter à force dans ces cupules.

**Patentansprüche**

1. Verfahren zur Bestimmung der Aktivität einer in den Behältern des Käseherstellung vorhandenen Substanz vom Bakteriophagtyp auf einem Mikroorganismus vom Milchsäurebakterientyp, gekennzeichnet durch die Folge der nachfolgenden Stufen:

(a) man stellt in einem Behälter ein lyophilisiertes Milieu her, das den Mikroorganismus und einen Teil eines Kurlturmediums diese Mikroorganismus enthält;

(b) man stellt eine Lösung her, die die Ergänzung dieses Kulturmediums enthält;

(c) man setzt zu dieser Lösung die besagte Substanz zu;

(d) man giesst die so erhaltene Flüssigkeit in den Behälter;

(e) man lässt den Behälter inkubieren; und

(f) man unterzieht den Behälter einem Test bezüglich der Gegenwart des Mikroorganismus.

2. Verfahren nach Anspruch 1 zur Bestimmung der Aktivität der besagten Substanz auf einem Gemisch von n Mikroorganismen, dadurch gekennzeichnet, dass die Stufe (a) durch die folgende Stufe (al) ersetzt wird:

(al) man stellt in jedem von n Behältern ein lyophilisiertes Milieu her, das einen betreffenden Mikroorganismus des Gemisches sowie einen Teil eines Kulturmediums der Gesamtheit der Mikroorganismen enthält, und wendet jede der Stufen (d), (e) und (f) auf jeden der Behälter an.

3. Verfahren nach Anspruch 1, dadurch gekennzeichnet, dass man im Falle, wo die besagte Substanz auch den Mikroorganismus oder das Mikroorganismengemisch enthält, zwischen den Stufen (a) oder (al) einerseits und (c) andererseits folgende zusätzliche Stufe ausführt:

(g) Man unterzieht die besagte Substanz einer Behandlung, die den Mikroorganismus oder das Mikroorganismengemisch entfernt; und das man die Stufe (c) durch die folgende Stufe ersetzt;

(cl) man setzt zu der besagten Lösung die durch die Stufe (g) behandelte Substanz zu.

4. Verfahren nach Anspruch 3, dadurch gekennzeichnet, dass die Behandlung der Stufe (g) in einem Erwärmen besteht.

5. Verfahren nach Anspruch 1, dadurch gekennzeichnet, dass man für den oder jeden Mikroorganismus in einem zusätzlichen Behälter das besagte lyophilisierte Milieu herstellt, eine Kontrollösung analog der besagten Lösung, aber zusätzlich einen Indikator der Aktivität des oder jeden Mikroorganismus enthaltend herstellt, die Kontrollösung in den zusätzlichen Behälter giesst und die Stufe (e) verlängert, bis der Indikator eine vorbestimmte Aktivität des oder jeden Mikroorganismus angezeigt hat.

6. Vorrichtung zur Durchführung des Verfahrens nach Anspruch 2, dadurch gekennzeichnet, dass sie
– einen Träger von Mikrotitrationsbechern (1), der n Reihen (9A, 9B . . .) von
– m Bechern (8) vereinigt, wobei jede Reihe ein lyophilisiertes Milieu enthält, das selbst einen betreffenden Mikroorganismus des Gemisches enthält,
– 4 (m-1) sterile Entnahmeröhren (3) und
– 4 m Verdünnungsröhren (4), die die Lösung enthalten, umfasst.

7. Vorrichtung nach Anspruch 6, dadurch gekennzeichnet, dass jede Reihe (9A, 9B . . .) des Becherträgers (1) einen (m)ten Becher (8) aufweist und dass eine (m)te Verdünnungsröhre (4) vorgesehen ist, die die besagte Lösung enthält, wobei eine Kontrollröhre mit den (m)ten Bechern verbunden ist.

8. Vorrichtung nach Anspruch 6, dadurch gekennzeichnet, dass sie ausserdem eine Röhre (6) eines Farbindikators in Anwesenheit der Mikroorganismen des Gemisches aufweist.

9. Vorrichtung nach Anspruch 6, dadurch gekennzeichnet, dass jede Verdünnungsröhre (4) einen Farbindikator je Aktivität der Mikroorganismen des Gemisches enthält.

10. Vorrichtung nach Anspruch 6, dadurch gekennzeichnet, dass sie ausserdem einen Rührkamm (7) aufweist, dessen Zähne (14) so ausgebildet sind, dass sie gleichzeitig in alle Behälter (8) ein und derselben Reihe (9A, 9B . . .) oder ein und derselben Kolonne (10a, 10b . . .) eindringen.

11. Vorrichtung nach Anspruch 6, dadurch gekennzeichnet, dass jede Becherreihe (9) mit einem Deckel (2) versehen ist, der aus einem Bogen besteht, auf welchem zum unteren Ende hin Blindkammern (12) in gleicher Zahl wie jene der Becher (8) und in solcher Ausbildung, dass sie unter Zwang in die Becher sich einpassen, vorspringen.

**Claims**

1. Method for detecting the activity of a bacteriophagous substance present in cheese dairy vats on a micro-organism of lactic bacteria, characterised by the sequence of the following steps:
(a) a freeze-dried medium containing the micro-organism and a part of a medium of the culture of this micro-organism is prepared in a receptacle;
(b) a solution containing the complement of the said culture medium is prepared;
(c) the said substance is added to this solution;
(d) the liquid thus obtained is poured into the receptacle;
(e) the receptacle is incubated; and
(f) the receptacle is subjected to a test for presence of the micro-organism.

2. Method according to claim 1, for determining the activity of the said substance on a mixture of n micro-organisms, characterised in that step (a) is substituted by the following step (al):
(al) a freeze-dried medium containing a respective micro-organism of the mixture as well as part of a medium of the culture of the ensemble of micro-organisms is prepared in each of n receptacles; and in that each of steps (d), (e) and (f) is applied to each of the receptacles.

3. Method according to claim 1, characterised in that in the case where the said substance also contains the micro-organism or the mixture of micro-organisms, the following additional step is effected, between, on the one hand. steps (a) or (al), and on the other hand, (c):
(g) the said substance is subjected to a treatment which eliminates from it the micro-organism or the mixture of micro-organisms;
and in that step (c) is substituted by the following step:
(cl) said substance treated by step (g) is added to the said solution.

4. Method according to claim 3, characterised in that the treatment of step (g) is constituted by heating.

5. Method according to claim 1, characterised in that for the or for each of the micro-organisms, the said freeze-dried medium is prepared in an additional receptacle; an analogous test solution to the said solution, but containing furthermore an indicator of the activity of the or each micro-organism, is prepared; the test solution is poured into the said additional receptacle and step (e) is continued until the said indicator has shown a predetermined activity of the or of each micro-organism.

6. Device for putting into operation a method according to claim 2, characterised in that it comprises:
– a holder for microtitration cupules (1) joining n rows (9A, 9B . . .) of
– m cupules (8), each row containing a freeze-dried medium which itself contains a respective micro-organism of the said mixture;
– 4 (m-1) sterile sampling tubes (3); and
– 4 m diluting tubes (4) containing the said solution.

7. Device according to claim 6, characterised in that each row (9A, 9B . . .) of the holder for cupules (1) consists of an (m)th cupule (8) and in that a provided (m)th diluting tube (4) containing the said solution, forming an indicator tube is connected to the (m)th cupules.

8. Device according to claim 6, characterised in that it comprises furthermore a tube (6) of a coloured indicator of the presence of the micro-organisms of the mixture.

9. Device according to claim 6, characterised in that each diluting tube (4) contains a coloured indicator of the activity of the micro-organisms of the mixture.

10. Device according to claim 6, characterised in that it comprises furthermore an agitating comb (7) the teeth of which (14) are adapted for penetrating simultaneously into all the receptacles (8) of one same row (9A, 9B . . .) or of one same column (10a, 10b . . .).

11. Device according to claim 6, characterised in that each row of cupules (9) is equipped with a cover (2) consisting of a sheet on which a number of mock cavities (12), equal to that of cupules (8), and adapted for fitting into these cupules with force, project towards the bottom.

FIG.1

FIG.2

FIG.3

FIG.4

FIG.5